# EUROPÄISCHE PATENTSCHRIFT

(11) **EP 0 728 451 B1**
(45) Veröffentlichungstag und Bekanntmachung des Hinweises auf die Patenterteilung: **03.05.2000**
(21) Anmeldenummer: 96102228.2
(22) Anmeldetag: 15.02.1996
(51) Int. Cl.: A61F 2/68

(54) **Bremskniegelenk**
Braked knee joint
Articulation du genou freinée

(30) Priorität: 24.02.1995 DE 19506426
(43) Veröffentlichungstag der Anmeldung: 28.08.1996
(73) Patentinhaber: Otto Bock Orthopädische Industrie Besitz- und Verwaltungs-Kommanditgesellschaft, 37115 Duderstadt (DE)
(72) Erfinder: Wagner, Helmut, D-37115 Duderstadt (DE); Krukenberg, Manfred, 37115 Duderstadt (DE)
(74) Vertreter: Gramm, Werner, Prof. Dipl.-Ing.

(56) Entgegenhaltungen:
- EP-A- 0 314 668
- EP-A- 0 325 347
- DE-A- 1 931 889
- DE-U- 9 320 853
- GB-A- 762 695
- US-A- 2 667 644

## Beschreibung

Die Erfindung betrifft ein Bremskniegelenk für eine Beinprothese.

Die US-Patentschrift 2,667,644 offenbart ein derartiges Bremskniegelenk für eine Beinprothese, mit einem Gelenkoberteil, einem Gelenkunterteil, einer drehfest mit einem Gelenkteil verbundenen Gelenkachse und einem ein Gelenkmittelteil bildenden Schwinghebel. Die bei diesem Bremskniegelenk vorgesehene Bremseinrichtung weist eine geschlossene, die Gelenkachse über ihren Umfang zumindest teilweise konzentrisch umschließende, mit Hydrauliköl befüllte Verdrängerkammer auf, die in zwei Kammern unterteilt ist, die über eine Ölleitung miteinander verbunden sind, die durch ein Ventil ganz oder teilweise verschließbar ist. Die Steuerung dieses Ventils erfolgt mechanisch über ein bis in den Kunstfuß geführtes Gestänge, dessen Beaufschlagung in Abhängigkeit von der Fußbelastung erfolgt.

Der Erfindung liegt die Aufgabe zugrunde, bei diesem vorbekannten Bremskniegelenk unter Beibehaltung einer natürlichen Gangphase für die durch Fußbelastung angesteuerte Bremseinrichtung eine verbesserte Konstruktion vorzusehen.

Diese Aufgabe wird erfindungsgemäß gelöst durch ein Bremskniegelenk für eine Beinprothese, mit einem Gelenkoberteil, einem Gelenkunterteil, einer drehfest mit einem Gelenkteil verbundenen Gelenkachse und einem ein Gelenkmittelteil bildenden Schwinghebel, der mit seinem streckseitigen Ende an einer parallel, ventral und distal zur Gelenkachse liegenden Schwingachse festgelegt ist und mit seinem beugeseitigen Ende die Gelenkachse umschließt, und mit einer durch Fußbelastung angesteuerten Bremseinrichtung, die eine geschlossene, die Gelenkachse über ihren Umfang zumindest teilweise konzentrisch umschließende, vorzugsweise mit Hydrauliköl befüllte Verdrängerkammer aufweist, die in dem zugeordneten Ende des Gelenkmittelteils angeordnet und von der als Drehkolben ausgebildeten, drehfest mit dem Gelenkoberteil verbundenen Gelenkachse in eine Streckkammer und eine Beugekammer unterteilt ist, die über eine Ölleitung miteinander verbunden sind, die durch einen Ventilkolben ganz oder teilweise verschließbar ist, der in dem Gelenkmittelteil gegen die Wirkung einer ihn in seine Offenstellung drückende Ventilkolbenfeder verschiebbar gelagert ist und bei Verschwenkung des Gelenkmittelteils in Beugerichtung um die im Gelenkunterteil angeordnete Schwingachse von einer im Gelenkunterteil vorgesehenen Verstelleinrichtung in seine Schließstellung gedrückt wird.

Dabei ist es vorteilhaft, wenn zur Schwungphasensteuerung in die die Streckkammer mit der Beugekammer verbindende Ölleitung zwei entgegengesetzt wirkende Drosselrückschlagventile in Reihe geschaltet sind.

Vorzugsweise vorgesehen ist ferner ein als Vorbringer dienendes Pleuel, das mit seinem einen Ende am Gelenkoberteil und seinem distalen Ende an einem im Gelenkunterteil untergebrachten Federspanner angelenkt ist, der von einer Vorbringerfeder beaufschlagt ist.

Um bei der Extension (Streckung) einen harten Anschlag zu vermeiden, kann erfindungsgemäß eine Endlagendämpfung vorgesehen sein. Vorzugsweise ist vorgesehen, daß zur Erzeugung einer Endlagendämpfung bei Extension die die Streckkammer mit der Beugekammer verbindende Ölleitung über einen Anschluß so an die Streckkammer angeschlossen ist, daß der Drehkolben diesen Anschluß in dem letzten Bereich seiner Drehbewegung in Richtung Extension zunehmend verschließt. Der genannte letzte Bereich der Drehbewegung kann vorzugsweise 10° - 15° betragen. Bei zunehmender Extension (Streckung) drückt der Drehkolben das Öl aus der Streckkammer über eine Verbindungsleitung in die Beugekammer. Durch den zunehmenden Verschluß dieser Ölauslaßleitung durch den Drehkolben wird ein die Streckdämpfung bewirkender Drosseleffekt hervorgerufen. Dabei kann der Verlauf der Dämpfung durch die am Anschluß vorbeigeführte Außenkontur des Drehkolbens bestimmt werden.

Um bei einer Umkehr der Drehrichtung des Drehkolbens aus der Extension in die Flexion (Beugung) einen von der Endlagendrossel hervorgerufenen Strömungswiderstand zu vermeiden, kann erfindungsgemäß vorgesehen werden, daß in den Anschluß der Ölleitung an die Beugekammer ein Rückschlagventil vorgesehen ist, das bei Einleitung der Beuge von dem aus der Beugekammer verdrängten Öl geöffnet wird.

Weitere Merkmale der Erfindung sind Gegenstand der Unteransprüche und werden in Verbindung mit weiteren Vorteilen der Erfindung anhand von Ausführungsbeispielen näher erläutert.

In der Zeichnung sind einige als Beispiele dienende Ausführungsformen der Erfindung dargestellt. Es zeigen:
- Figur 1: in Seitenansicht ein Kniegelenk mit Unterschenkelprothese;
- Figur 2: in Seitenansicht ein sich in Streckstellung befindliches Bremskniegelenk mit hydraulischer Ansteuerung;
- Figur 3: das Bremskniegelenk gemäß Figur 4 in Beugestellung;
- Figur 4: die Darstellung gemäß Figur 4 im Längsschnitt;
- Figur 5: die Darstellung gemäß Figur 5 im Längsschnitt;
- Figur 6: einen hydraulischen Strömungsplan;
- Figur 7: eine schematische Detaildarstellung der justierbaren Rückschlagventile für die Schwungphasensteuerung des Bremskniegelenkes;
- Figur 8: eine abgewandelte Ausführungsform in einer Darstellung gemäß Figur 6 und
- Figur 9: das Bremskniegelenk gemäß Figur 10 in Beugestellung.

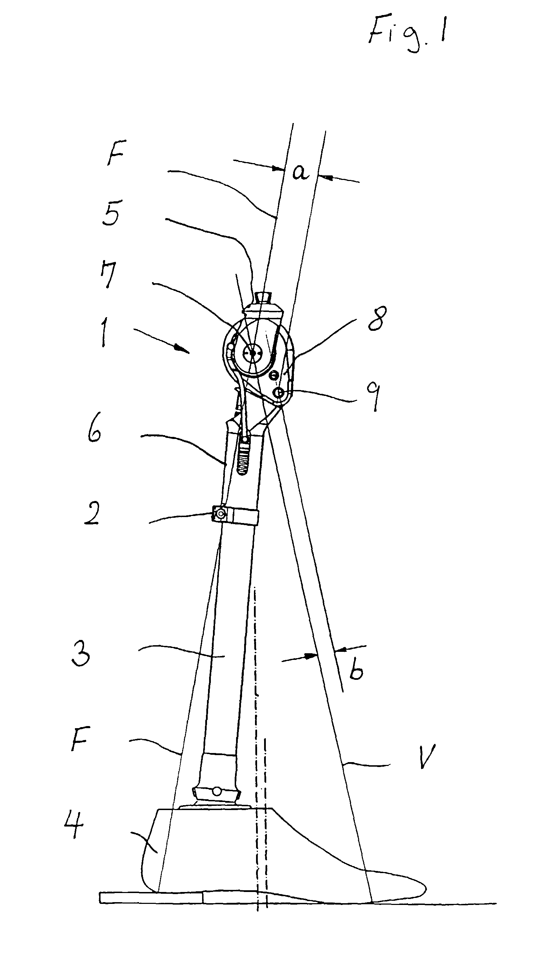

Figur 1 zeigt ein Bremskniegelenk 1, an dessen distalen Ende über eine Klemmverbindung 2 ein den Unterschenkel der Prothese bildendes Rohr 3 befestigt ist, das an seinem unteren Ende mit einem Prothesenfuß 4 verbunden ist.

Das Bremskniegelenk 1 setzt sich zusammen aus einem Gelenkoberteil 5, einem Gelenkunterteil 6, einer Gelenkachse 7 sowie aus einem durch Fußbelastung beaufschlagbaren, ein Gelenkmittelteil 8 bildenden Schwinghebel, der um eine parallel zur Gelenkachse 7 liegende Schwingachse 9 verschwenkbar ist.

Figur 1 läßt erkennen, daß die Schwingachse 9 ventral und distal zur Gelenkachse 7 angeordnet ist.

Eingezeichnet sind in Figur 1 eine die Fersenbelastung symbolisierende Kraftlinie F sowie eine die Vorfußbelastung symbolisierende Kraftlinie V, die beide durch die Gelenkachse 7 geführt sind. Das Bremsmoment, das der um die Schwingachse 9 verschwenkbare Schwinghebel 8 auf die Gelenkachse 7 auszuüben vermag, wird bestimmt durch den jeweiligen Hebelarm, der sich aus dem Stand der jeweiligen Belastungslinie von der Schwingachse 9 ergibt. Figur 1 läßt erkennen, daß der sich für die Fersenbelastungslinie F ergebende Hebelarm a größer ist als der sich für die Vorfußbelastungslinie V ergebende Hebelarm b. Dies ist eine Folge der Tieferlegung der Schwingachse 9 gegenüber der Gelenkachse 7 und bewirkt, daß bei Fersenbelastung ein maximales Bremsmoment aufgebracht wird, während dieses Bremsmoment bei Vorfußbelastung erheblich geringer ist und dadurch eine entsprechende Beugeeinleitung für die Gangphase ermöglicht. Würden die beiden Achsen 7, 9 in derselben Horizontalebene liegen, ergäben sich für die Fersen- und Vorfußbelastung annähernd gleich große Bremsmomente, wodurch die Beugeeinleitung für die Gangphase beeinträchtigt würde.

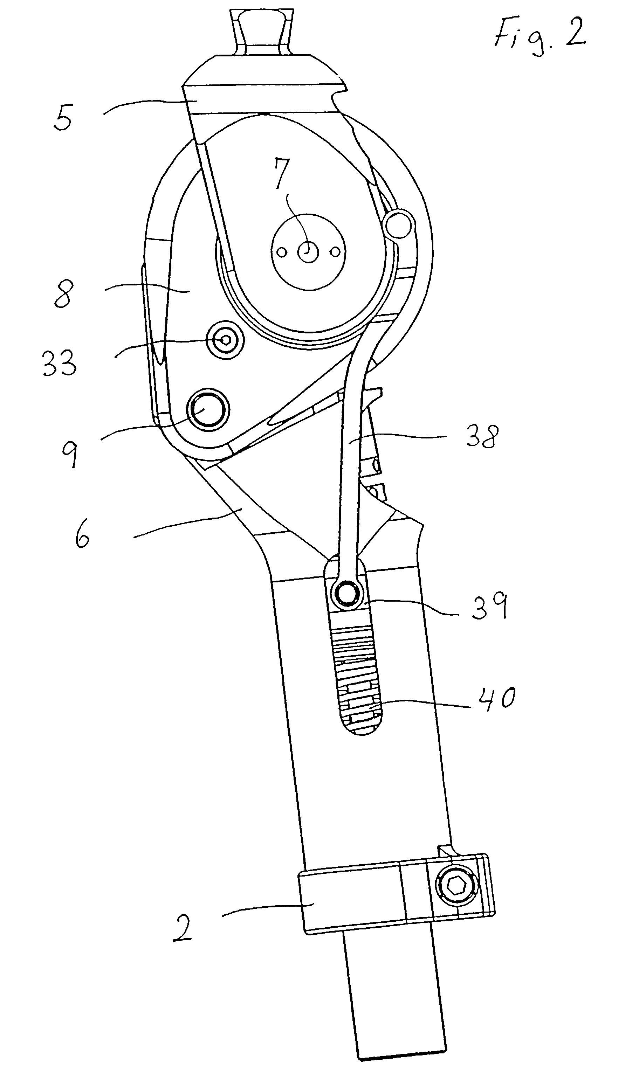

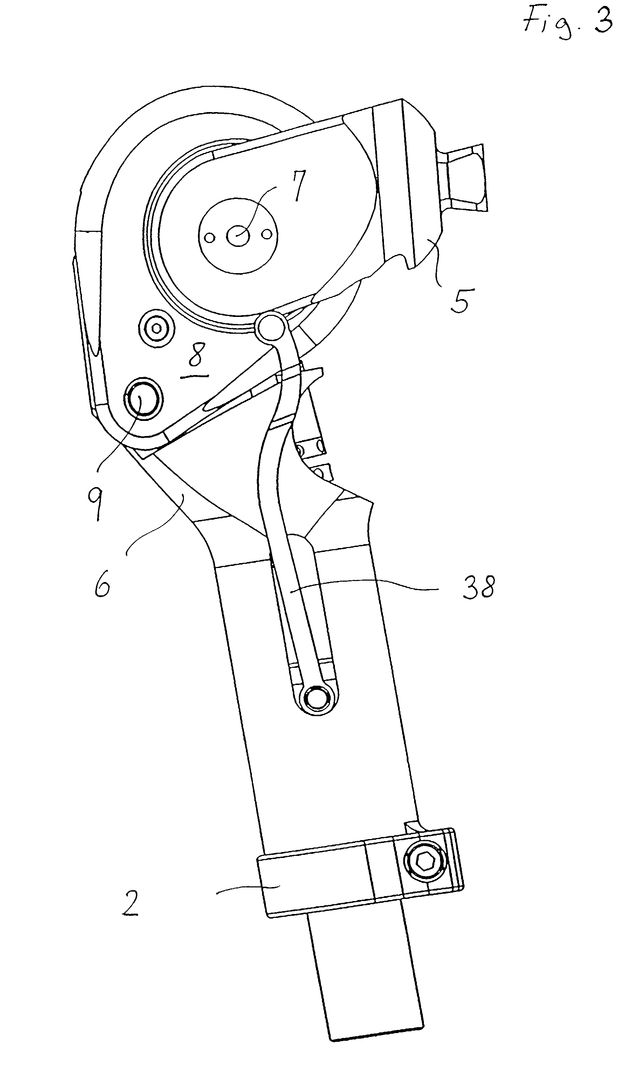

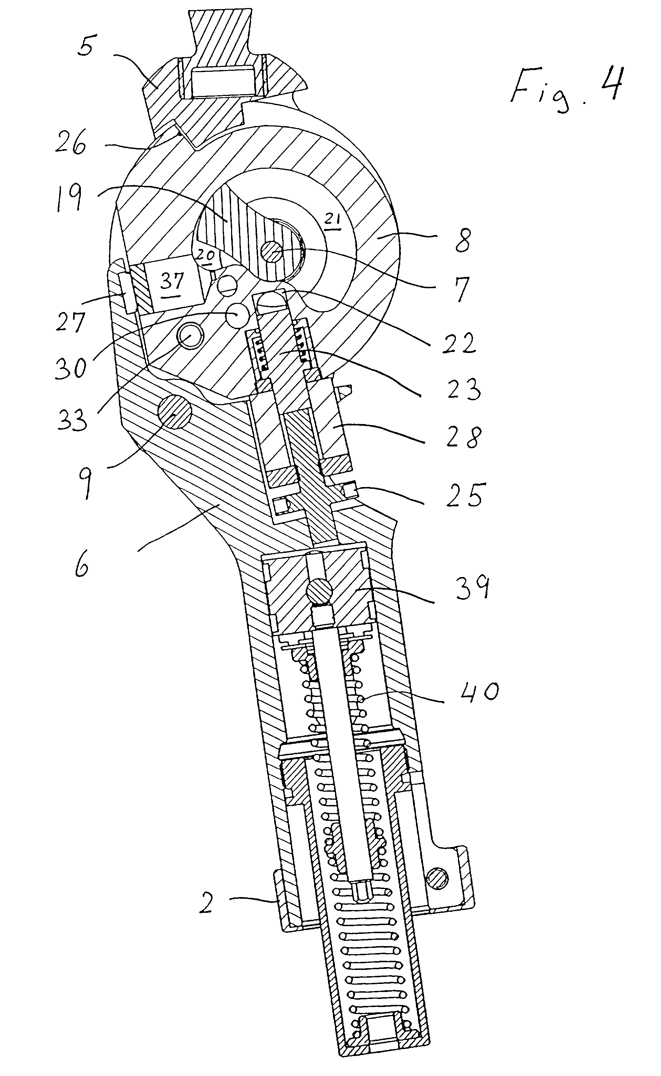

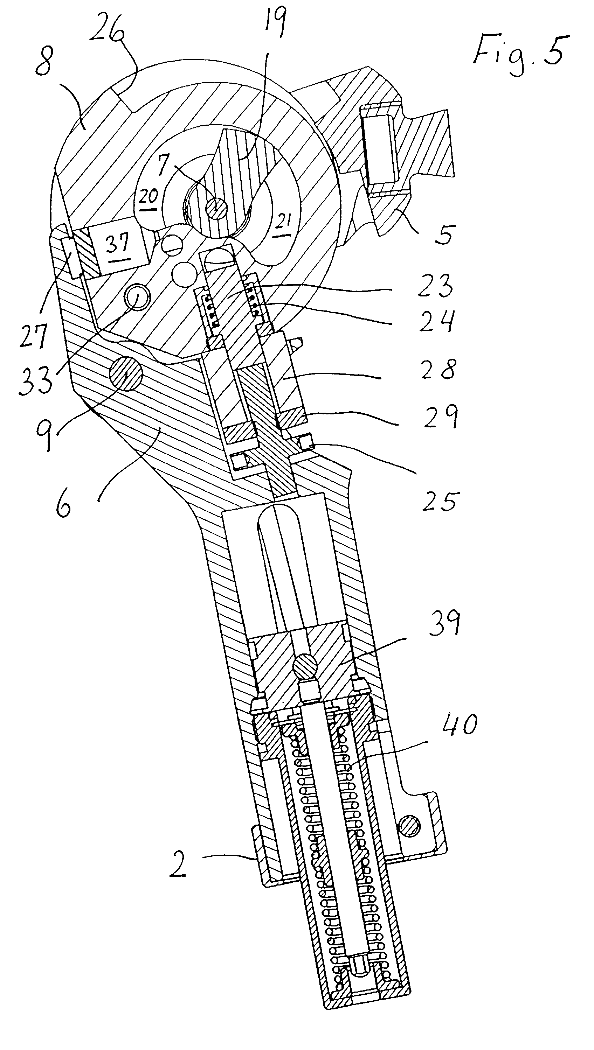

Die Figuren 2 bis 5 zeigen ein hydraulisch angesteuertes Bremskniegelenk. Hier ist die Gelenkachse 7 über ihren Umfang zumindest teilweise konzentrisch umschlossen von einer vorzugsweise mit Hydrauliköl befüllten Verdrängerkammer, die in dem ein Gelenkmittelteil bildenden Schwinghebel 8 angeordnet und von der als Drehkolben 19 ausgebildeten, drehfest mit dem Gelenkoberteil 5 verbundenen Gelenkachse 7 in eine Streckkammer 20 und eine Beugekammer 21 unterteilt ist, die über eine Ölleitung 22 miteinander verbunden sind. Letztere ist durch einen Ventilkolben 23 ganz oder teilweise verschließbar, der in dem Gelenkmittelteil 8 gegen die Wirkung einer ihn in seine Offenstellung drükkenden Ventilkolbenfeder 24 verschiebbar gelagert ist und bei Verschwenkung des Gelenkmittelteils 8 in Beugerichtung von einer im Gelenkunterteil 6 vorgesehenen Verstelleinrichtung in seine Schließstellung gedrückt wird. Diese Verstelleinrichtung besteht aus einer im Gelenkunterteil 6 gelagerten, manuell verdrehbaren Justierschraube 25, die das distale Ende des Ventilkolbens 23 beaufschlagt.

Am Gelenkmittelteil 8 ist ein Streckanschlag 26 für das Gelenkoberteil 5 vorgesehen. Die Drehbewegung des auf der Schwingachse 9 gelagerten Gelenkmittelteils 8 in dessen Streckstellung wird durch einen am Gelenkunterteil 6 sitzenden Streckanschlag 27 und in dessen Beugestellung durch einen federelastischen, ebenfalls am Gelenkunterteil 6 festgelegten Beugeanschlag 28 begrenzt, der durch eine Gummihülse gebildet ist, die den Ventilkolben 23 und dessen Justierschraube 25 teilweise umgreift. Dieser federelastische Beugeanschlag 28 dient zur Umschaltung von der Schwungphase in die Standphase und kann durch eine das distale Ende der Gummihülse beaufschlagende, auf der Justierschraube 25 geführte Justiermutter 29 auf eine an den Patienten angepaßte Vorspannung eingestellt werden.

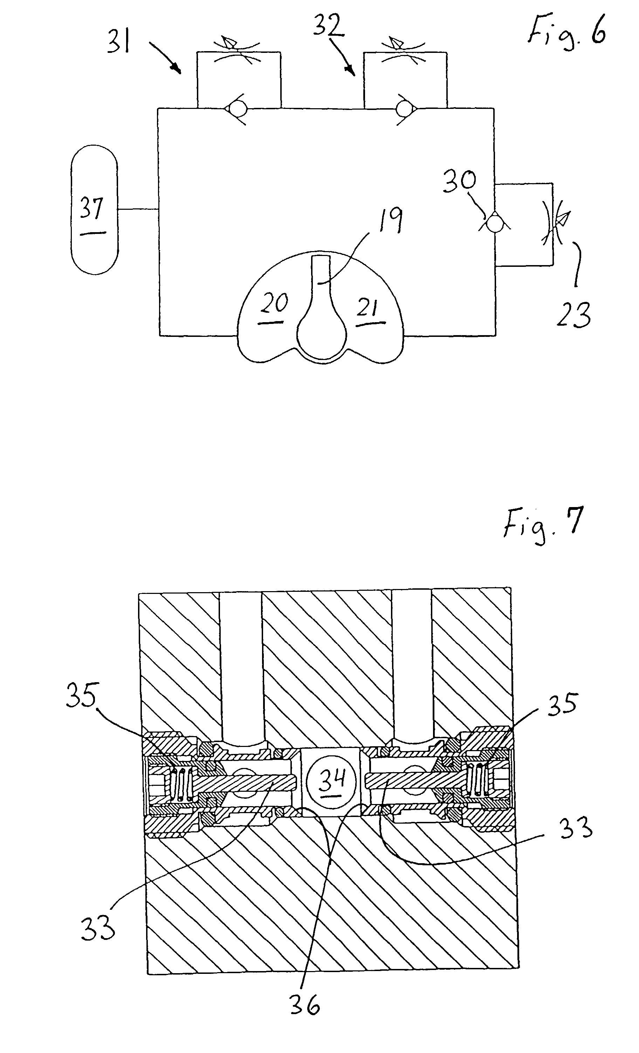

In der die beiden Kammern 20, 21 miteinander verbindenden Ölleitung 22 ist ein die durch den Ventilkolben 23 gebildete Drosselstelle umgehendes Rückschlagventil 30 vorgesehen, das bei der Streckung des Gelenkes und dem dadurch bedingten Ölfluß von der Streckkammer in die Beugekammer öffnet (siehe auch Figur 6). Dadurch wird verhindert, daß das bei der Streckung des Gelenkes verdrängte Hydrauliköl durch den Ventilkolben 23 beeinflußt wird.

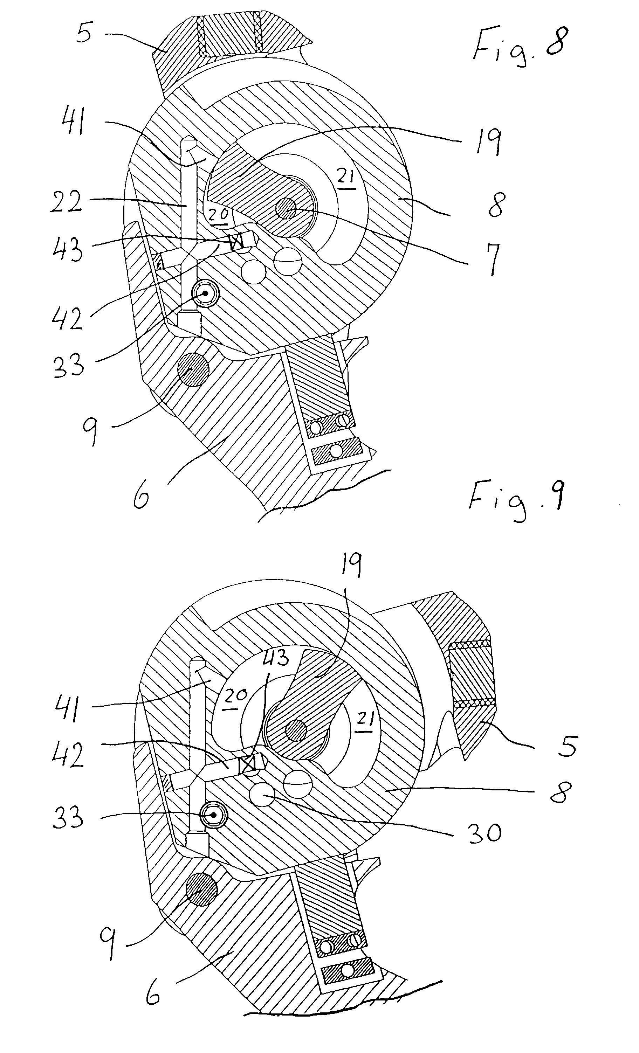

Um die Schwungphase steuern zu können, sind in die vorstehend genannte Ölleitung 22 zwei entgegengesetzt wirkende Drosselrückschlagventile 31, 32 in Reihe geschaltet (siehe Figuren 6 u. 7), die über je einen manuell von außen längsverstellbaren Justierstift 33 beaufschlagbar sind. Figur 7 zeigt, daß die beiden Rückschlagventile 31, 32 eine gemeinsame Kugel 34 aufweisen, die bei entsprechender Druckbeaufschlagung zwischen den beiden Justierstiften 33 verschiebbar ist. Die Druckbeaufschlagung der Kugel 34 erfolgt durch den sie umströmenden Ölfluß. Mit steigendem Ölfluß nimmt der auf die Kugel 34 wirkende Druck zu, wodurch die Kugel 34 gegen die Wirkung einer Feder 35 näher an den entsprechenden Ventilsitz 36 verschoben wird. Hierdurch ergibt sich eine höhere Drosselung des Ölflusses. Der Widerstand (Dämpfung) nimmt somit bei schnellerer Kniebewegung zu.

An die Verdrängerkammer 20, 21 ist ein Volumenänderungen des Hydrauliköls ausgleichender Druckspeicher 37 angeschlossen.

Ferner ist ein als Vorbringer dienendes Pleuel 38 vorgesehen, das mit seinem einen Ende am Gelenkoberteil 5 und mit seinem distalen Ende an einem im Gelenkunterteil 6 untergebrachten Federspanner 39 angelenkt ist, der von einer Vorbringerfeder 40 beaufschlagt ist.

Bei der in den Figuren 8 und 9 dargestellten Ausführungsform ist eine Endlagendämpfung bei Extension vorgesehen. Die die Streckkammer 20 mit der Beugekammer 21 verbindende Ölleitung 22 ist über einen Anschluß 41 so an die Streckkammer 20 angeschlossen, daß der Drehkolben 19 diesen Anschluß 41 in dem letzten Bereich seiner entgegen dem Uhrzeigersinn durchgeführten Drehbewegung in Richtung Extension zunehmend verschließt. Dadurch wird eine Drosselwirkung erzeugt, die die Streckdämpfung bewirkt. Der Verlauf der Dämpfung hängt dabei von der am Anschluß 41 vorbeigeführten Außenkontur des Drehkolbens 19 ab. In den Anschluß 42 der Ölleitung 22 an die Beugekammer 21 ist ein Rückschlagventil 43 eingesetzt, das bei Einleitung der Beuge von dem aus der Beugekammer 21 verdrängten Öl geöffnet wird.

## Patentansprüche

1. Bremskniegelenk für eine Beinprothese, mit einem Gelenkoberteil (5), einem Gelenkunterteil (6), einer drehfest mit einem Gelenkteil verbundenen Gelenkachse (7) und einem ein Gelenkmittelteil (8) bildenden Schwinghebel, der mit seinem streckseitigen Ende an einer parallel, ventral und distal zur Gelenkachse (7) liegenden Schwingachse (9) festgelegt ist und mit seinem beugeseitigen Ende die Gelenkachse (7) umschließt, und mit einer durch Fußbelastung angesteuerten Bremseinrichtung, die eine geschlossene, die Gelenkachse (7) über ihren Umfang zumindest teilweise konzentrisch umschließende, vorzugsweise mit Hydrauliköl befüllte Verdrängerkammer (20, 21) aufweist, die in dem zugeordneten Ende des Gelenkmittelteils (8) angeordnet und von der als Drehkolben (19) ausgebildeten, drehfest mit dem Gelenkoberteil (5) verbundenen Gelenkachse (7) in eine Streckkammer (20) und eine Beugekammer (21) unterteilt ist, die über eine Ölleitung (22) miteinander verbunden sind, die durch einen Ventilkolben (23) ganz oder teilweise verschließbar ist, der in dem Gelenkmittelteil (8) gegen die Wirkung einer ihn in seine Offenstellung drückende Ventilkolbenfeder (24) verschiebbar gelagert ist und bei Verschwenkung des Gelenkmittelteils (8) in Beugerichtung um die im Gelenkunterteil (6) angeordnete Schwingachse (9) von einer im Gelenkunterteil (6) vorgesehenen Verstelleinrichtung in seine Schließstellung gedrückt wird.

2. Bremskniegelenk nach Anspruch 1, **dadurch gekennzeichnet**, daß die genannte Verstelleinrichtung eine manuell verdrehbare Justierschraube (25) ist, die das distale Ende des Ventilkolbens (23) beaufschlagt.

3. Bremskniegelenk nach Anspruch 1 oder 2, **dadurch gekennzeichnet**, daß am Gelenkmittelteil (8) ein Streckanschlag (26) für das Gelenkoberteil (5) vorgesehen ist.

4. Bremskniegelenk nach Anspruch 1, 2 oder 3, **dadurch gekennzeichnet**, daß die Drehbewegung des auf der Schwingachse (9) gelagerten Gelenkmittelteils (8) in dessen Streckstellung durch einen am Gelenkunterteil (6) sitzenden Streckanschlag (27) und in dessen Beugestellung durch einen federelastischen, ebenfalls am Gelenkunterteil (6) festgelegten Beugeanschlag (28) begrenzt ist.

5. Bremskniegelenk nach Anspruch 4, **dadurch gekennzeichnet,** daß der federelastische Beugeanschlag (28) durch eine manuell betätigbare Justiereinrichtung vorspannbar ist.

6. Bremskniegelenk nach Anspruch 2 und 5, **dadurch gekennzeichnet**, daß der federelastische Beugeanschlag (28) eine Gummihülse ist, die den Ventilkolben (23) und dessen Justierschraube (25) teilweise umgreift.

7. Bremskniegelenk nach Anspruch 6, **dadurch gekennzeichnet**, daß die Justiereinrichtung für den Beugeanschlag (28) eine auf der genannten Justierschraube (25) geführte, das distale Ende des Beugeanschlags (28) beaufschlagende Justiermutter (29) ist.

8. Bremskniegelenk nach einem der Ansprüche 1 bis 7, **dadurch gekennzeichnet**, daß in der genannten Ölleitung (22) ein die durch den Ventilkolben (23) gebildete Drosselstelle umgehendes Rückschlagventil (30) vorgesehen ist, das bei der Streckung des Gelenkes und dem dadurch bedingten Ölfluß von der Streckkammer (20) in die Beugekammer (21) öffnet.

9. Bremskniegelenk nach einem der Ansprüche 1 bis 8, **dadurch gekennzeichnet**, daß zur Schwungphasensteuerung in die die Streckkammer (20) mit der Beugekammer (21) verbindende Ölleitung (22) zwei entgegengesetzt wirkende Drosselrückschlagventile (31, 32) in Reihe geschaltet sind.

10. Bremskniegelenk nach Anspruch 9, **dadurch gekennzeichnet,** daß zur manuellen Justierung der Schwungphasensteuerung die Rückschlagventile (31, 32) über je einen Justierstift (33) beaufschlagbar sind.

11. Bremskniegelenk nach Anspruch 10, **dadurch gekennzeichnet,** daß die beiden Rückschlagventile (31, 32) eine gemeinsame Kugel (34) aufweisen, die zwischen den beiden Justierstiften (33) verschiebbar ist.

12. Bremskniegelenk nach Anspruch 11, **dadurch gekennzeichnet,** daß zumindest einer der Justierstifte (33) bei entsprechendem Druck der genannten Kugel (34) gegen die Wirkung einer Feder (35) in Richtung auf den Ventilsitz (36) verschiebbar ist.

13. Bremskniegelenk nach einem der Ansprüche 1 bis 12, **dadurch gekennzeichnet,** daß an die Verdrängerkammer (20, 21) ein Volumenänderungen des Hydrauliköles ausgleichender Druckspeicher (37) angeschlossen ist.

14. Bremskniegelenk nach einem der Ansprüche 1 bis 13, **gekennzeichnet** durch ein als Vorbringer dienendes Pleuel (38), das mit seinem einen Ende am Gelenkoberteil (5) und mit seinem distalen Ende an einem im Gelenkunterteil (6) untergebrachten Federspanner (39) angelenkt ist, der von einer Vorbringerfeder (40) beaufschlagt ist.

15. Bremskniegelenk nach einem der Ansprüche 1 bis 14, **dadurch gekennzeichnet**, daß zur Erzeugung einer Endlagendämpfung bei Extension die die Streckkammer (20) mit der Beugekammer (21) verbindende Ölleitung (22) über einen Anschluß (41) so an die Streckkammer (20) angeschlossen ist, daß der Drehkolben (19) diesen Anschluß (41) in dem letzten Bereich seiner Drehbewegung in Richtung Extension zunehmend verschließt.

16. Bremskniegelenk nach Anspruch 15, **dadurch gekennzeichnet,** daß in den Anschluß (42) der Ölleitung (22) an die Beugekammer (21) ein Rückschlagventil (43) eingesetzt ist, das bei Einleitung der Beuge von dem aus der Beugekammer (21) verdrängten Öl geöffnet wird.

## Claims

1. Braking knee joint for an artificial leg, having a joint upper part (5), a joint lower part (6), a joint axle (7) which is connected non-rotatably to a joint part, and a rocking lever forming a joint middle part (8), which is fixed by its extension-side end to a swing axle (9) lying parallel, ventrally and distally relative to the joint axle (7) and with its flexion-side end encloses the joint axle (7), and having a braking device, which is activated by foot load and comprises a closed displacement chamber (20, 21) which at least partially concentrically encloses the joint axle (7) over its periphery, is preferably filled with hydraulic oil, is disposed in the associated end of the joint middle part (8) and is subdivided by the joint axle (7), which takes the form of a rotary piston (19) and is non-rotatably connected to the joint upper part (5), into an extension chamber (20) and a flexion chamber (21), which are connected to one another by an oil line (22) which is totally or partially closable by a valve piston (23), which is supported in the joint middle part (8) so as to be displaceable counter to the action of a valve piston spring (24) pressing it into its open position and, upon swivelling of the joint middle part (8) in flexion direction about the swing axle (9) disposed in the joint lower part (6), is pressed into its closed position by an adjusting device provided in the joint lower part (6).

2. Braking knee joint according to claim 1, **characterized in that** said adjusting device is a manually rotatable adjusting screw (25) which acts upon the distal end of the valve piston (23).

3. Braking knee joint according to claim 1 or 2, **characterized in that** an extension stop (26) for the joint upper part (5) is provided on the joint middle part (8).

4. Braking knee joint according to claim 1, 2 or 3, **characterized in that** the rotational movement of the joint middle part (8) supported on the swing axle (9) is delimited in its extension position by an extension stop (27) seated on the joint lower part (6) and in its flexion position by a spring-elastic flexion stop (28) which is fixed likewise on the joint lower part (6).

5. Braking knee joint according to claim 4, **characterized in that** the spring-elastic flexion stop (28) is preloadable by means of a manually operable adjusting device.

6. Braking knee joint according to claim 2 and 5, **characterized in that** the spring-elastic flexion stop (28) is a rubber sleeve which partially embraces the valve piston (23) and the latter's adjusting screw (25).

7. Braking knee joint according to claim 6, **characterized in that** the adjusting device for the flexion stop (28) is an adjusting nut (29), which is guided on said adjusting screw (25) and acts upon the distal end of the flexion stop (28).

8. Braking knee joint according to one of claims 1 to 7, **characterized in that** provided in said oil line (22) is a check valve (30), which bypasses the throttle point formed by the valve piston (23) and opens upon extension of the joint and the resultant flow of oil from the extension chamber (20) into the flexion chamber (21).

9. Braking knee joint according to one of claims 1 to 8, **characterized in that**, for swing phase control, two oppositely acting throttle check valves (31, 32) are connected in series into the oil line (22) connecting the extension chamber (20) to the flexion chamber (21).

10. Braking knee joint according to claim 9, **characterized in that**, for manually adjusting the swing phase control, the check valves (31, 32) may be acted upon in each case by an adjusting pin (33).

11. Braking knee joint according to claim 10, **characterized in that** the two check valves (31, 32) have a common ball (34) which is displaceable between the two adjusting pins (33).

12. Braking knee joint according to claim 11, **characterized in that** at least one of the adjusting pins (33), when suitably pressed by said ball (34), is displaceable in the direction of the valve seat (36) counter to the action of a spring (35).

13. Braking knee joint according to one of claims 1 to 12, **characterized in that** connected to the displacement chamber (20, 21) is an accumulator (37) which compensates variations in volume of the hydraulic oil.

14. Braking knee joint according to one of claims 1 to 13, **characterized** by a connecting rod (38) serving as a bring-forward device, which is coupled by its one end to the joint upper part (5) and by its distal end to a spring-tensioning device (39), which is housed in the joint lower part (6) and acted upon by a bring-forward spring (40).

15. Braking knee joint according to one of claims 1 to 14, **characterized in that**, to produce an end-position damping upon extension, the oil line (22) connecting the extension chamber (20) to the flexion chamber (21) is connected by a connection (41) in such a way to the extension chamber (20) that the rotary piston (19) in the last region of its rotational movement in extension direction increasingly closes said connection (41).

16. Braking knee joint according to claim 15, **characterized in that** inserted into the connection (42) of the oil line (22) to the flexion chamber (21) is a check valve (43) which, when flexion is initiated, is opened by the oil displaced from the flexion chamber (21).

## Revendications

1. Articulation du genou freinée, pour prothèse de la jambe, comprenant une partie supérieure d'articulation (5), une partie inférieure d'articulation (6), un axe d'articulation (7) lié en rotation à une partie d'articulation, et un levier oscillant formant partie intermédiaire d'articulation (8), qui par son extrémité côté extension est fixé à un axe oscillant (9) situé parallèlement, en position ventrale et distale par rapport à l'axe d'articulation (7) et qui par son extrémité côté flexion entoure l'axe d'articulation (7), et comprenant un dispositif de freinage piloté par la charge supportée par le pied, lequel dispositif de freinage présente une chambre de refoulement (20, 21) fermée, remplie de préférence avec de l'huile hydraulique, entourant concentriquement l'axe d'articulation (7) sur au moins une partie de son pourtour, qui est aménagée dans l'extrémité correspondante de la partie intermédiaire d'articulation (8), chambre que l'axe d'articulation (7) formant piston rotatif (19) lié en rotation à la partie supérieure d'articulation (5) subdivise en une chambre d'extension (20) et une chambre de flexion (21), qui sont reliées l'une à l'autre par l'intermédiaire d'une conduite d'huile (22) pouvant être fermée totalement ou partiellement par un piston formant soupape (23) monté coulissant dans la partie intermédiaire d'articulation (8) contre l'action d'un ressort (24) du piston formant soupape, lequel ressort pousse le piston formant soupape dans sa position d'ouverture, le piston formant soupape étant poussé dans sa position de fermeture par un dispositif de réglage prévu dans la partie inférieure d'articulation (6) au cours du pivotement de la partie intermédiaire d'articulation (8) dans le sens de la flexion autour de l'axe d'oscillation (9) disposé dans la partie inférieure d'articulation (6).

2. Articulation du genou freinée selon la revendication 1, **caractérisée en ce que** ledit dispositif de réglage est une vis de réglage (25) que l'on peut tourner manuellement, qui agit sur l'extrémité distale du piston formant soupape (23).

3. Articulation du genou freinée selon la revendication 1 ou 2, **caractérisée en ce qu**'une butée d'extension (26) est prévue sur la partie intermédiaire d'articulation (8) pour la partie supérieure d'articulation (5).

4. Articulation du genou freinée selon la revendication 1, 2 ou 3, **caractérisée en ce que** le mouvement de rotation de la partie intermédiaire d'articulation (8) supportée sur l'axe d'oscillation (9), est limité dans la position d'extension de la partie intermédiaire d'articulation par une butée d'extension (27) placée sur la partie inférieure d'articulation (6) et dans sa position de flexion par une butée élastique de flexion (28) placée également sur la partie inférieure d'articulation (6).

5. Articulation du genou freinée selon la revendication 4, **caractérisée en ce que** la butée élastique de flexion (28) peut être précontrainte par un dispositif de réglage actionnable manuellement.

6. Articulation du genou freinée selon les revendications 2 et 5, **caractérisée en ce que** la butée élastique de flexion (28) est un manchon de caoutchouc, qui enveloppe partiellement le piston formant soupape (23) et sa vis de réglage (25).

7. Articulation du genou freinée selon la revendication 6, **caractérisée en ce que** le dispositif de réglage pour la butée de flexion (28) est un écrou de réglage (29) guidé sur ladite vis de réglage (25), agissant sur l'extrémité distale de la butée de flexion (28) .

8. Articulation du genou freinée selon l'une des revendications 1 à 7, **caractérisée en ce que** dans ladite conduite d'huile (22) est prévu un clapet anti-retour (30) contournant l'étranglement formé par le piston formant soupape (23), lequel clapet s'ouvre lors de l'extension de l'articulation et de l'écoulement d'huile qui en résulte de la chambre d'extension (20) dans la chambre de flexion (21).

9. Articulation du genou freinée selon l'une des revendications 1 à 8, **caractérisée en ce que** deux clapets anti-retour d'étranglement (31, 32) agissant en sens inverse sont disposés en série dans la conduite d'huile (22) qui relie la chambre d'extension (20) à la chambre de flexion (21), pour le contrôle des phases de balancement.

10. Articulation du genou freinée selon la revendication 9, **caractérisée en ce que** pour le réglage manuel du contrôle des phases de balancement, les clapets anti-retour (31, 32) peuvent être soumis chacun à l'action d'une goupille de réglage (33).

11. Articulation du genou freinée selon la revendication 10, **caractérisée en ce que** les deux clapets anti-retour (31, 32) présentent une bille commune (34) capable de se déplacer entre les deux goupilles de réglage (33).

12. Articulation du genou freinée selon la revendication 11, **caractérisée en ce que** l'une au moins des goupilles de réglage (33) est capable de se déplacer dans la direction du siège de soupape (36) lors d'une pression correspondante de ladite bille (34), contre l'action d'un ressort (35).

13. Articulation du genou freinée selon l'une des revendications 1 à 12, **caractérisée en ce qu**'un accumulateur de pression (37) compensateur des variations de volume de l'huile hydraulique est raccordé à la chambre de refoulement (20, 21).

14. Articulation du genou freinée selon l'une des revendications 1 à 13, **caractérisée par** une bielle (38) servant d'avanceur, qui est articulée par une première extrémité à la partie supérieure d'articulation (5) et par son extrémité distale à un tenseur de ressort (39) inséré dans la partie inférieure d'articulation (6), tenseur sur lequel agit un ressort d'avanceur (40).

15. Articulation du genou freinée selon l'une des revendications 1 à 14, **caractérisée en ce que** pour produire un amortissement de fin de course à l'extension, la conduite d'huile (22) qui relie la chambre d'extension (20) à la chambre de flexion (21) est raccordée à la chambre d'extension (20) par l'intermédiaire d'un raccord (41) de telle sorte que le piston rotatif (19) obture de plus en plus ce raccord (41) dans la dernière phase de son mouvement de rotation dans le sens de l'extension.

16. Articulation du genou freinée selon la revendication 15, **caractérisée en ce que** dans le raccord (42) de la conduite d'huile (22) à la chambre de flexion (21) est placé un clapet anti-retour (43), qui est ouvert par l'huile chassée de la chambre de flexion (21) lors de l'initiation de la flexion.
